# EUROPEAN PATENT SPECIFICATION

(11) **EP 2 149 383 B1**
(45) Date of publication and mention of the grant of the patent: **13.03.2013**
(21) Application number: 09009807.0
(22) Date of filing: 29.07.2009
(51) Int. Cl.: A61M 5/32, B21G 1/08

(54) **Double-side coated syringe needle**
Doppelseitig beschichtete Spritzennadel
Aiguille pour seringue à double face

(30) Priority: 01.08.2008 TW 97129346; 22.06.2009 TW 98211176 U
(43) Date of publication of application: 03.02.2010
(73) Proprietor: Lin, Wen-Hung, East Dist. Tainan 70174 (TW)
(72) Inventor: Lin, Wen-Hung, East Dist. Tainan 70174 (TW)
(74) Representative: Stüven, Ralf

(56) References cited:
- EP-A1- 1 491 154
- JP-A- 9 279 331
- JP-A- 2006 000 521
- JP-A- 2007 181 638

## Description

### Background

This disclosure relates to a double-side coated syringe needle, more particularly to a zirconium or zirconia coated syringe needle.

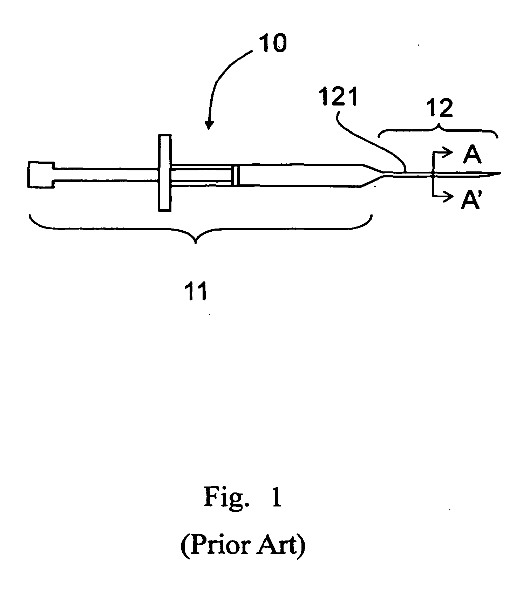

Fig.1 shows a traditional syringe needle. A medical syringe needle 10 has a syringe needle body 11 and a hollow stainless steel needle head 12. The hollow needle head 12 is used to pierce into human being's skin tissue to inject liquid medicine into a human body or withdraw tissue samples from the hollow metal needle head.

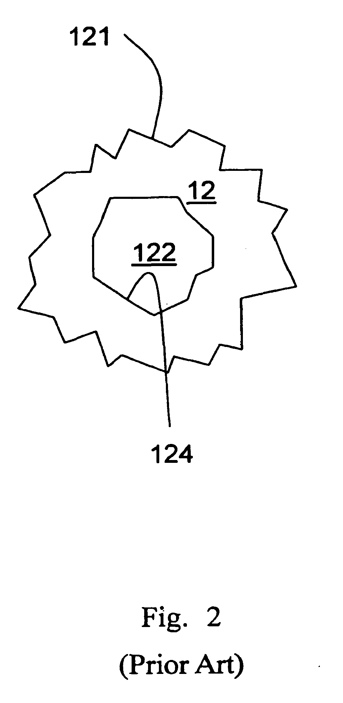

Fig. 2 shows a second view along line AA' of Fig. 1. The hollow metal needle head 12 has a hollow passage 122 and a rough surface 121 from a microcosmic view. Due to manufacturing process level and cost problem to a syringe needle 12, the roughness of the surface 121 of a traditional metal syringe needle head 10 is significant large enough to cause irregular tissue tear while piercing into human's body. The irregular tissue tear causes prolonged recover for a wound. In addition, a traditional syringe needle could make the wound tissue oxidation which causes the wound tissue being cured or repaired slowly by stem cells.

When hollow metal needle head 12 of the traditional syringe is used in human tissue biopsy, human tissue is taken out through the hollow passage 122. The known irregular and rough inner metal surface 124 tears the tissue, the sample integrity and consistency can not be maintained for different samples, and thereafter test data comparison accuracy can not be ensured over the samples. For a better medical study or observation over tissue samples, people need to get a smooth surface of syringe for tissue sampling to improve data accuracy of the samples.

A double-side coated syringe needle according to the preamble of claim 1 is disclosed in document EP 1 491 154.

### Brief Description of the Drawings

Fig. 1 shows a prior art syringe needle
Fig. 2 shows a section view of Fig. 1 along a section line AA'
Fig. 3 shows an arrangement outside the scope of the present invention
Fig. 4 shows an embodiment of the present invention.

### Detailed Description of Embodiments

The present invention overcomes the disadvantage of irregular tissue tear to which a traditional needle usually causes. According to this application, a syringe needle is coated with a smooth layer of zirconium or zirconia over both its inner surface and outer surface. The coating makes a hollow metal syringe needle to be with a smooth inner surface and outer surface.

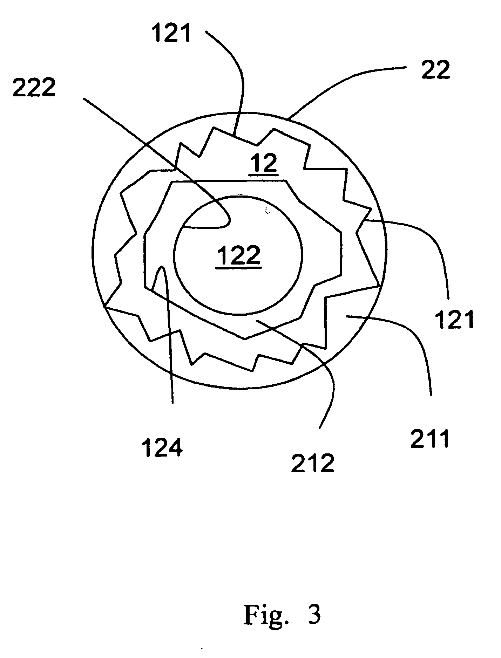

Fig. 3 shows an arrangement outside the scope of the present invention. A section view of a double-side coated hollow metal needle head 12 shows a first smooth layer 211 with a thickness of 0.1-1000 µm is applied over the outer rough surface 121 of the hollow metal needle head 12 to flat the roughness to yield an ideal outer smooth surface 22.

A second smooth layer 212 with a thickness of 0.1-1000 µm is applied over the rough inner surface 124 of the hollow metal needle head 12 to flat the inner surface roughness to yield an ideal inner smooth surface 222. The central hollow 122 can be used to withdraw body tissues with perfect peripheral tissue for study or observation.

The outer smooth layer 211 is a material of zirconium or zirconia. The final outer smooth layer 211 can be made from vacuum plating or immersed chemical deposition to form a smooth surface 22 over the inner surface and outer surface of the hollow metal needle head 12.

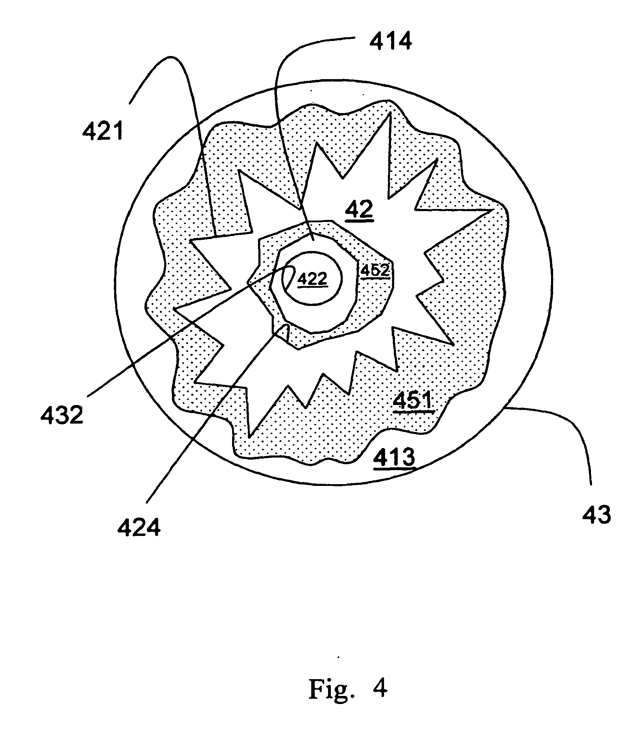

Fig. 4 shows an embodiment of the present invention. A first intermediate layer 451 is configured in between the hollow metal needle head 42 and an outer smooth layer 413. The outer intermediate layer 451 is used for flatting a badly uneven outer surface 421. The outer intermediate layer 451 is applied to fill into the valleys to reduce the topography difference between valleys and peaks so as to provide a smoother outer surface of the hollow metal needle head 42 before applying a zirconium or zirconia fine layer 413 over the outer surface to yield a final smooth outer surface 43.

The material of the intermediate smooth layer 451 is selected from a material containing carbon content higher than that in a stainless steel. The intermediate smooth layer 451 also plays a role of a barrier layer for preventing carbon diffusion from the hollow stainless needle head 42 into the fine smooth layer 413. Carbon diffusion from the hollow metal needle head 42 might cause the smooth layer 413 deteriorated. The material of the intermediate layer 451 is selected from a group consisting of Teflon, PP, PE, PS, PET, PEEK, PMMA, and Nylon each of which has a thermal expansion coefficient between that of a stainless steel and zirconia. The coefficient is 10* 10⁻⁶-14* 10⁻⁶/°C for a stainless steel, and 3 * 10⁻⁶-9* 10⁻⁶/°C for zirconia.

There are several methods which can be used for coating the intermediate layer 451 over the surface of the hollow metal needle head 42 such as immersed chemical deposition or die casting.

The final outer smooth layer 413 can be made through vacuum plating or immersed chemical deposition and forming a smooth outer surface 43 over the surface of the intermediate layer 451. Similarly, a second intermediate layer 452 is configured in between the hollow metal needle head 42 and the smooth layer 414 for yielding a smooth inner surface 432 surrounding a passing hole 422.

Each of the smooth layers 211, 212, 413, 414 is exemplified as a material of zirconium or zirconia , actually the material which can be used for the smooth layer 21, 212, 413, 414 is one selected from a group consisting of: aluminum, silicon, silicon dioxide, aluminum oxide, titanium, titanium oxide, molybdenum, niobium, and tantalum. While an embodiments has been described by way of example, it will be apparent to those skilled in the art that various modifications may be made without departing from the scope of the present invention, as defined by the appended claims.

## Claims

1. A double-side coated syringe needle, comprising:
a hollow metal needle head (42), having an inner surface (424) and an outer surface (421);
a first ceramic layer (414), coated on the inner surface (424) of the hollow metal; and
a second ceramic layer (413), coated on the outer surface (421) of the hollow metal **characterised in that** the needle further comprises
a first intermediate layer (451), configured in between the hollow metal and the second ceramic layer (413); and
a second intermediate layer (452), configured in between the hollow metal and the first ceramic layer (414),
wherein the metal is stainless steel and the material of the first intermediate layer (451) has a higher carbon content than the stainless steel, the first intermediate layer (451) being a barrier layer for preventing carbon diffusion from the hollow stainless needle head (42) into the second ceramic layer (413).

2. A needle as claimed in claim 1, wherein the ceramic layer (413, 414) comprises at least one material selected from a group consisting of: aluminum, aluminum oxide, silicon, silicon dioxide, zirconium, zirconia (zirconium oxide), titanium, titanium oxide, molybdenum, niobium, and tantalum.

3. A needle as claimed in any of claims 1 or 2, wherein the intermediate layer (451, 452) is a material selected from a group consisting of Teflon, PP, PE, PS, PET, PEEK, PMMA, and Nylon.

4. A needle as claimed in any of the preceding claims, wherein a thermal expansion coefficient for the intermediate layer (451, 452) is in the range between that of the hollow metal and zirconia.

5. A needle as claimed in any of the preceding claims, wherein a thermal expansion coefficient for the intermediate layer (451, 452) is in the range between 10*10⁻⁶-14*10⁻⁶/°C and 3*10⁻⁵-9*10⁻⁶/°C depending on materials selected.

## Patentansprüche

1. Beidseitig beschichtete Spritzennadel, umfassend:
einen hohlen Metalhiadelkopf (42), der eine innere Oberfläche (424) und eine äußere Oberfläche (421) aufweist;
eine erste keramische Schicht (414), die auf die innere Oberfläche (424) des hohlen Metalls aufgebracht ist; und
eine zweite keramische Schicht (413), die auf die äußere Oberfläche (421) des hohlen Metalls aufgebracht ist,
**dadurch gekennzeichnet, dass** die Nadel ferner
eine erste Zwischenschicht (451), die zwischen dem hohlen Metall und der zweiten keramischen Schicht (413) angeordnet ist, und
eine zweite Zwischenschicht (452), die zwischen dem hohlen Metall und der ersten keramischen Schicht (414) angeordnet ist, umfasst,
wobei das Metall Edelstahl ist und das Material der ersten Zwischenschicht (451) einen höheren Kohlenstoffgehalt als der Edelstahl aufweist, und wobei die erste Zwischenschicht (451) eine Sperrschicht zur Verhinderung einer Kohlenstoffdiffusion aus dem hohlen Edelstahlnadelkopf (42) in die zweite keramische Schicht (413) ist.

2. Nadel nach Anspruch 1, wobei die keramische Schicht (413, 414) mindestens ein Material umfasst, das aus der Gruppe ausgewählt ist, bestehend aus: Aluminum, Aluminumoxid, Silizium, Siliziumdioxid, Zirconium, Zirconia (Zirconiumoxid), Titan, Titanoxid, Molybdän, Niob und Tantal.

3. Nadel nach einem der Ansprüche 1 oder 2, wobei die Zwischenschicht (451, 452) ein Material ist, das ausgewählt ist aus der Gruppe, bestehend aus Teflon, PP, PE, PS, PET, PEEK, PMMA und Nylon.

4. Nadel nach einem der vorhergehenden Ansprüche, wobei ein Wärmeäusdehnungskoeffizient für die Zwischenschicht (451, 452) im Bereich zwischen dem des hohlen Metalls und Zirconia liegt.

5. Nadel nach einem der vorhergehenden Ansprüch, wobei ein Wärmeausdehnungskoeffizient für die Zwischenschicht (451, 452) in Abhängigkeit von den gewählten Materialien im Bereich zwischen 10*10⁻⁶-14*10⁻⁶/°C und 3*10⁻⁶-9*10⁻⁶/°C liegt.

## Revendications

1. Aiguille de seringue enduite sur deux côtés, comprenant :
une tête d'aiguilles de métal creux (42), ayant une surface interne (424) et une surface externe (421) ;
une première couche de céramique (414), enduite sur la surface interne (424) du métal creux ; et
une seconde couche de céramique (413), enduite sur la surface externe (421) du métal creux, **caractérisée en ce que** l'aiguille comprend en outre
une première couche intermédiaire (451), configurée entre le métal creux et la seconde couche de céramique (413) ; et
une seconde couche intermédiaire (452), configurée entre le métal creux et la première couche de céramique (414),
dans laquelle le métal est un acier inoxydable et le matériau de la première couche intermédiaire (451) possède une plus forte teneur en carbone que l'acier inoxydable, la première couche intermédiaire (451) étant une couche barrière pour empêcher la diffusion du carbone de la tête de l'aiguille en acier inoxydable creux (42) dans la seconde couche de céramique (413).

2. Aiguille selon la revendication 1, dans laquelle la couche de céramique (413, 414) comprend au moins un matériau choisi dans le groupe constitué par : l'aluminium, l'oxyde d'aluminium, le silicium, le dioxyde de silicium, le zirconium, la zircone (oxyde de zirconium), le titane, l'oxyde de titane, le molybdène, le niobium et le tantale.

3. Aiguille selon la revendication 1 ou 2, dans laquelle la couche intermédiaire (451, 452) est un matériau choisi dans le groupe constitué par le téflon, le PP, le PE, le PS, le PET, le PEEK, le PMMA et le nylon.

4. Aiguille selon l'une quelconque des revendications précédentes, dans laquelle un coefficient d'expansion thermique pour la couche intermédiaire (451, 452) est compris entre celui du métal creux et de la zircone.

5. Aiguille selon l'une quelconque des revendications précédentes, dans laquelle un coefficient d'expansion thermique pour la couche intermédiaire (451, 452) est compris dans la plage entre 10*10-6-14*10-6/°C et 3*10 6-9*10-6/°C en fonction des matériaux choisis.
